(19) **Europäisches Patentamt**
**European Patent Office**
**Office européen des brevets**

(11) **EP 4 376 943 B1**

(12) **EUROPEAN PATENT SPECIFICATION**

(45) Date of publication and mention
of the grant of the patent:
**22.07.2026  Bulletin 2026/30**

(51) International Patent Classification (IPC):
*A61N 1/36* (2006.01)          *A61N 5/06* (2006.01)
*A61N 5/067* (2006.01)         *A61N 1/05* (2006.01)

(21) Application number: **22850500.4**

(22) Date of filing: **27.07.2022**

(52) Cooperative Patent Classification (CPC):
**A61N 5/0601; A61N 1/36017; A61N 1/36021;**
**A61N 1/3603; A61N 1/36062; A61N 1/36071;**
**A61N 5/0622; A61N 5/067;** A61N 1/0551;
A61N 2005/0612; A61N 2005/0626;
A61N 2005/063; A61N 2005/0659;
A61N 2005/0662; A61N 2005/0666

(86) International application number:
**PCT/US2022/074215**

(87) International publication number:
**WO 2023/010055 (02.02.2023 Gazette 2023/05)**

(54) **FULL-DUPLEX IPG SYSTEM AND ELECTRO-OPTICAL PERCUTANEOUS LEAD**

VOLLDUPLEX-IPG-SYSTEM UND ELEKTROOPTISCHE PERKUTANE LEITUNG

SYSTÈME IPG DUPLEX INTÉGRAL ET FIL PERCUTANÉ ÉLECTRO-OPTIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **27.07.2021   US 202163203649 P**

(43) Date of publication of application:
**05.06.2024   Bulletin 2024/23**

(73) Proprietor: **Wavegate Corporation**
**Lake Charles, LA 70605 (US)**

(72) Inventors:
• **WOLF, Erich, W., II**
**Lake Charles, LA 70605 (US)**
• **O'NEAL, Dennis, Patrick**
**Lake Charles, LA 70605 (US)**

(74) Representative: **Forresters IP LLP**
**Skygarden**
**Erika-Mann-Straße 11**
**80636 München (DE)**

(56) References cited:
US-A- 6 159 165          US-A1- 2010 030 300
US-A1- 2011 295 344      US-A1- 2013 317 572
US-A1- 2014 005 755      US-A1- 2017 071 474
US-A1- 2018 326 219      US-A1- 2020 192 279
US-A1- 2021 001 114      US-A1- 2021 001 114

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## FIELD OF THE INVENTION

The present invention relates to an improved implantable pulse generator ("IPG") and header combination using optical reflectometry in spinal cord stimulation ("SCS"). The invention provides an implantable pulse generator systems according to claims 1 and 7. Embodiments of the invention are defined in the dependent claims.

## BACKGROUND OF THE INVENTION

Chronic pain may arise from a variety of conditions, most notably from nerve injury as in the case of neuropathic pain, or from chronic stimulation of mechanical nociceptors such as with spinal pain. Functional ability may be severely impacted by pain, which often is refractory to pharmacological and surgical treatment. In such cases, SCS can be an effective treatment for pain by modulating physiological transmission of pain signals from the periphery to the brain. This may be achieved by applying electrical impulses to the spinal cord via an electrode array implanted adjacent the spinal canal.

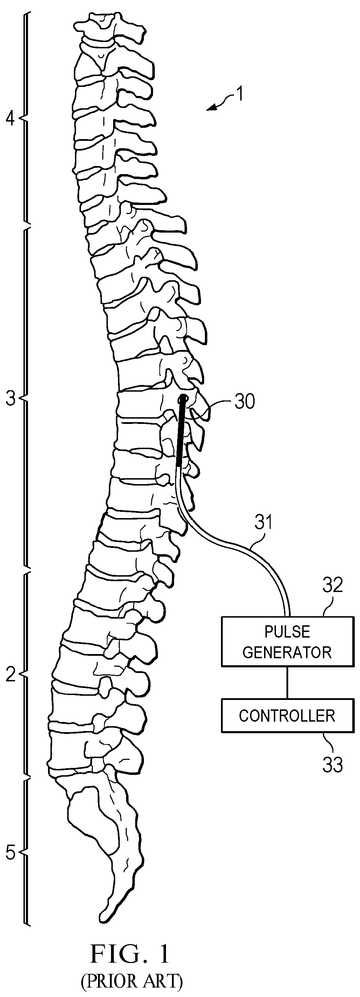

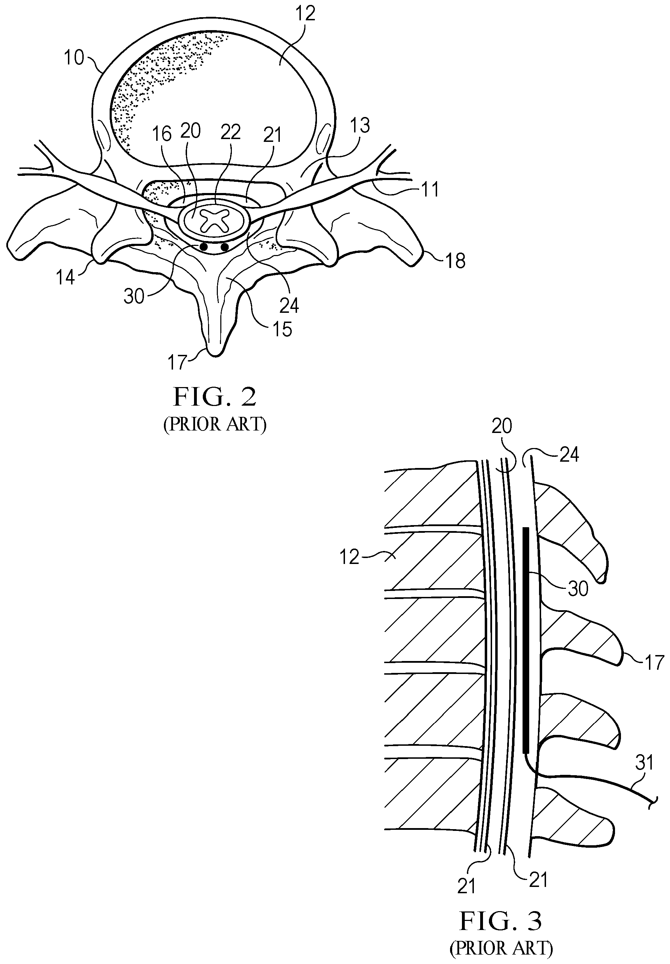

Referring to Figures 1, 2 and 3, a typical IPG system of the prior art will be described. Spinal column **1** is shown to have a number of vertebrae, categorized into four sections or types: lumbar vertebrae **2,** thoracic vertebrae **3,** cervical vertebrae **4** and sacral vertebrae **5.** Cervical vertebrae **4** include the 1st cervical vertebra (C1) through the 7th cervical vertebra (C7). Just below the 7th cervical vertebra is the first of twelve thoracic vertebrae **3** including the 1st thoracic vertebra (T1) through the 12th thoracic vertebra (T12). Just below the 12th thoracic vertebrae **3,** are five lumbar vertebrae **2** including the 1st lumbar vertebra (L1) through the 5th lumbar vertebra (L5), the 5th lumbar vertebra being attached to sacral vertebrae **5** (S1 to S5), sacral vertebrae **5** being naturally fused together in the adult.

Representative vertebra **10,** a thoracic vertebra, is shown to have a number of notable features which are in general shared with lumbar vertebrae **2** and cervical vertebrae **4.** The thick oval segment of bone forming the anterior aspect of vertebra **10** is vertebral body **12.** Vertebral body **12** is attached to bony vertebral arch **13** through which spinal nerves **11** run. Vertebral arch **13,** forming the posterior of vertebra **10,** is comprised of two pedicles **14,** which are short stout processes that extend from the sides of vertebral body **12** and bilateral laminae **15.** The broad flat plates that project from pedicles **14** join in a triangle to form a hollow archway, spinal canal **16.** Spinous process **17** protrudes from the junction of bilateral laminae **15.** Transverse processes **18** project from the junction of pedicles **14** and bilateral laminae **15.** The structures of the vertebral arch protect spinal cord **20** and spinal nerves **11** that run through the spinal canal.

Surrounding spinal cord **20** is dura **21** that contains cerebrospinal fluid (CSF) **22.** Epidural space **24** is the space within the spinal canal lying outside the dura.

One or more electrodes **30** are positioned in epidural space **24** between dura **21** and the walls of spinal canal **16** towards the dorsal aspect of the spinal canal nearest bilateral laminae **15** and spinous process **17.** Electrode **30** has electrode leads **31** which are connected to IPG **32** and controller **33.**

IPG **32** provides the electrical stimulation in the form of current pulses to the spinal cord through lead **31** to electrode **30.** The pulses generate an electric field. The electric field impinges on targeted neurons of the spinal cord and disrupts the perception of pain. The amplitude of the electrical field is critical to success of spinal cord stimulation. An inadequate electric field will fail to depolarize the targeted neurons, rendering the treatment ineffective. An excess electric field stimulates neighboring cell populations which results in a noxious stimulation.

Establishing a consistent, therapeutic, and non-noxious level of stimulation is predicated upon establishing an ideal current density within the spinal cord's targeted neurons. Fundamentally, this should be a simple matter of establishing an optimal electrode current given the local bulk conductivity of the surrounding tissues. But in practice, the optimal electrode current changes as a function of patient position and activity due to motion of the spinal cord as the spinal cord floats in cerebrospinal fluid within the spinal canal. Significant changes in distance between the epidural electrode array and the targeted spinal cord neurons have been shown to occur. Consequently, optimal stimulation requires dynamic adjustment of the electrode stimulating current as a function of distance between the electrode array and the spinal cord.

Dynamic modulation of spinal cord stimulator electrode current as a function of distance between the electrode array and the spinal cord thus has several benefits. Excess stimulation current can be avoided, thus reducing the prospects of noxious stimulation and potentially reducing device power consumption. Inadequate stimulation current can also be avoided, thus eliminating periods of compromised therapeutic efficacy.

Dynamic modulation of electrode current can be controlled through the use of optical reflectometry to determine the thickness of the dorsal cerebrospinal fluid (dCSF) column between the spinal cord and the electrode array. An optical signal is transmitted into the surrounding tissue and collected by a sensor to calculate the approximate distance between the electrode and the spinal cord. The stimulus magnitude is modified accordingly to provide the optimal current for pain relief. Examples of this technology are shown in U.S. Patent Nos. 10,035,019 and 9,656,097, both to Wolf II, and both incorporated herein by reference.

One challenge, to IPG implantation and usage is package size. Long-term survival of the IPG in the in vivo environment, mitigates that the package size be as small as possible to reduce rejection rates and to lower scarring times. A small package size also decreases the possibility of migration of the package and the surgical leads which emanate from it.

Another challenge to IPG systems is proper interpretation of the reflected optical signal. Half duplex systems of the prior art require that multiple surgical leads be precisely placed by the targeted neurons. Such placement during surgery is difficult. The problem is further exacerbated by lead migration, which may disalign the fibers and degrade optical feedback which controls modulation of the stimulation signal.

Yet another challenge to IPG systems is power constraints and heat generation. Power usage of the IPG must be kept to a minimum in order to assure long term battery life of the IPG package. Further, heat generation in the in vivo environment must be minimized to prevent rejection. Therefore, power consumption and heat generation both must be as low as possible.

Yet another challenge to optical IPG systems is Fresnel reflections. Fresnel reflections occur at all optical interfaces of the lead and through fiber and can be assumed to account for about 4% signal loss per optical interface. Interface losses are generally constant over time. Fiber degradation caused by bending also produces Fresnel losses. However, fiber degradation losses are not constant and change over time. Optical losses effect the accuracy of the optical feedback which controls the stimulation signal.

Another challenge to optical IPG systems is that laser output changes due to power variations and due to part age, thereby altering the characteristics of the optical system over time. The changes in the optical system reduce the effectiveness of the stimulation signal control and so reduce the efficacy of the stimulation signal.

The prior art has attempted to address these challenges in a number of ways, yet all have fallen short.

For example, U.S. Patent No. 9,656,097 to Wolf, II describe a full duplex IPG lead, which allows both a transmit ray and receive ray to travel down the same fiber. However, *Wolf* discloses use of a circulator to separate the two rays. A circulator is not practical because of the size of the circulator package and because of the optical losses incurred.

As another example, U.S. Patent No. 7,742,817 to Malinowski, et al. describes an IPG with connectors for electrical leads and an epoxy coating for biocompatibility. However, *Malinowski* does not disclose the use of optical feedback to achieve proper stimulation signal strength.

As another example, U.S. Publication No. 2021/0001114 to Wolf, II, discloses coupling of an optical lead to an IPG header adjacent a ruby passthrough window, vertically aligned with a photo detector. However, *Wolf* fails to disclose a way to recognize or compensate for optical system degradation over time.

U.S. Publication No. 2018/0154152 to Chabrol discloses a system for deep brain stimulation using a probe with stimulation electrodes and a light emitting optical fiber. However, *Chabrol* fails to address using the light signal to control a stimulation signal to the spinal cord. *Chabrol* also fails to recognize optical signal degradation over time or a way to test for resulting losses.

Deficiencies exist in the prior art related to power usage constraints, heat generation, and maintaining accurate optical feedback over time. Thus, there is a need in the art for an improved IPG including header orientation, connectors, leads and electrodes which provide a stable optical signal while tracking optical signal degradation due to Fresnel reflections and reducing power consumption and heat generation.

## BRIEF DESCRIPTION OF THE DRAWINGS

In the detailed description of the preferred embodiments presented below, reference is made to the accompanying drawings.

Figure 1 is a side view of the human spine showing the approximate position of a percutaneous lead and IPG for spinal cord stimulation.
Figure 2 shows an axial view of a thoracic vertebra indicating the position of the spinal cord and a percutaneous lead pair.
Figure 3 shows a sagittal cross-sectional view of the human spine showing the approximate position of a percutaneous lead.
Figure 4 shows a schematic diagram of an IPG system of a preferred embodiment.
Figure 5 is an isometric view of a preferred IPG device.
Figure 6 is an exploded isometric view of a preferred IPG device.
Figure 7 is an exploded isometric view of a preferred IPG case.
Figure 8 is an exploded isometric view of a preferred IPG header.
Figure 9 is an exploded isometric view of a preferred die stack.
Figure 10 is a partial cross-section view of a preferred header assembly.
Figure 11 is an isometric view of a lead assembly of a preferred embodiment.
Figure 12 is a cross-section view of a lead assembly of a preferred embodiment.
Figure 13 is a flowchart of a preferred control program for operation of the IPG.
Figure 14 is a flowchart of a preferred method of Fresnel compensation.
Figure 15 is a flowchart of a preferred method of normalizing source emission.
Figure 16 is a flowchart of a preferred method of correcting for source power variation.

## DETAILED DESCRIPTION OF THE INVENTION

In the description that follows, like parts are marked throughout the specification and figures for the same numerals. The figures are not necessarily drawn to scale and may be shown in exaggerated or generalized form in the interest of clarity and conciseness. Unless otherwise noted, all tolerances and uses of the term "about" indicate plus or minus 5%.

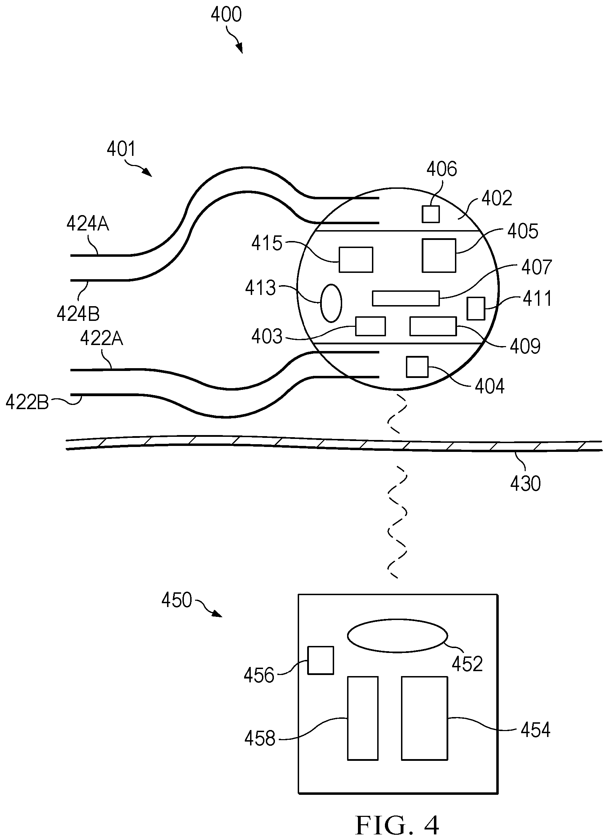

Referring then to Figure 4, preferred embodiment of stimulation system **400** will be further described. Stimulation system **400** further comprises IPG **401** in operative communication with controller **450**.

IPG **401** is housed in hermetically sealed composite case **402**, as will be further described. Composite case **402** houses the operative components of the IPG and serves to anchor leads **422A, 422B, 424A** and **424B**, , as will be further described.

The operative components of the system comprise optical folding assembly **406**, optically aligned with leads **424A** and **424B**. Similarly, the operative components include optical folding assembly **404**, optically aligned with leads **422A** and **422B**. Optical folding assembly **406** sends and receives optical signals from leads **424A** and **424B** to be interpreted by optical signal processor **405**. Likewise, optical folding assembly **404** sends and receives optical signals from leads **422A** and **422B** to be interpreted by optical signal processor **403**. Optical signal processor **405** and optical signal processor **403** are operatively connected to main processor **407**, which controls the functions of the IPG, as will be further described. Main processor **407** is operatively connected to signal generator **409**, which generates electrical stimulation signals which are transmitted through leads **424A, 424B, 422A** and **422B** to the spinal cord to targeted nerve populations, as will be further described. Communications circuit **411** is also operatively connected to main processor **407**. Main processor **407** receives programming instructions and control signals from the communications circuit, as will be further described.

IPG **401** includes battery **415**. Battery **415** is operatively connected to all the electrical components of the system. Battery **415** receives recharging current from induction coil **413**, as will be further described.

IPG **401** is surgically implanted beneath dermis **430**.

Controller **450** exists outside dermis **430**. Controller **450** includes main processor **454**, which controls the functions of the controller. Main processor **454** is connected to I/O keyboard and display unit **458**, which is fixed in the exterior casing. Main processor **454** is operatively connected to communications circuit **456**. Communications circuit **456** is wirelessly connected to communications circuit **411**. Main processor **454** includes sufficient memory to receive instructions from I/O keyboard and display unit **458** and transfer them through communications circuit **456** and communications circuit **411** to main processor **407** for controlling the operation of IPG **401**. Main processor **454** is further operatively connected to induction coil **452**. Induction coil **452** is inductively coupled to induction coil **413** which transfers power for the charging of the battery.

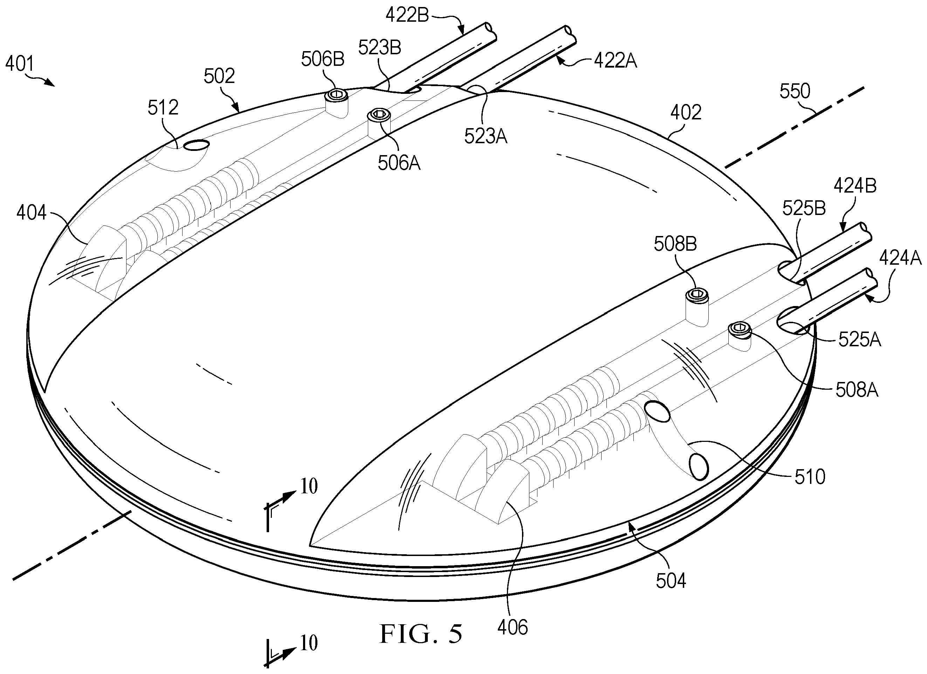

Referring then to Figure 5, IPG **401** will be further described.

Composite case **402** mechanically supports header assembly **502** and header assembly **504,** which are both positioned on opposite sides of the IPG, parallel to a single lateral axis **550**. Positioning the header assemblies at opposite sides of the case is important because it improves the dispersion of the heat generated by the lasers, as will be further described. Each header assembly preferably is manufactured from a transparent epoxy resin which serves to fix the optical and electrical components in place.

Header assembly **502** includes optical folding assembly **404**. Optical folding assembly **404** is optically aligned with lead retainer holes **523A** and **523B**. Leads **422A** and **422B** are positioned in and optically aligned by lead retainer holes **523A** and **523B**. Leads **422A** and **422B** are removably secured in the header by virtue of set screws **506A** and **506B,** respectively. The leads abut optical folding assembly **404,** as will be further described.

Header assembly **502** further comprises tie down portal **512**, for use in securing the IPG to the fascia during surgery.

Header assembly **504** includes optical folding assembly **406**. Optical folding assembly **406** is optically aligned with lead retainer holes **525A** and **525B**. Leads **424A** and **424B** are positioned in and optically aligned by lead retainer holes **525A** and **525B**. Leads **424A** and **424B** are removably secured in the header by virtue of set screws **508A** and **508B,** respectively. The leads abut optical folding assembly **406,** as will be further described.

Header assembly **504** further comprises tie down portal **510**, for use in securing the IPG to the fascia during surgery.

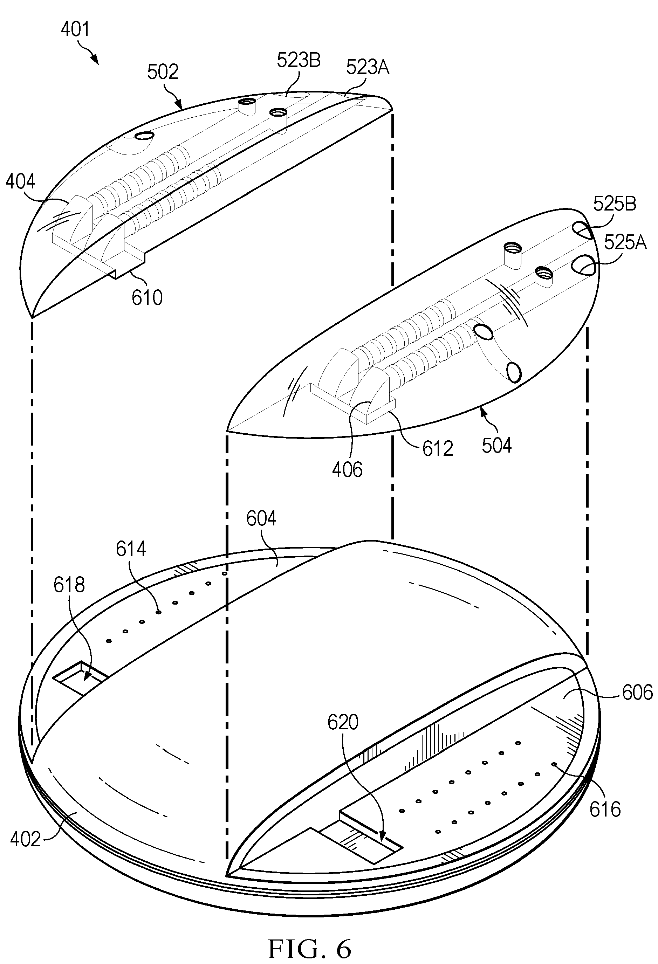

Referring then to Figure 6, IPG **401** will be further described.

Composite case **402** further comprises header bay **604** and header bay **606**. Each header bay is an angular indention in the composite case. The header bays are generally parallel and diametrically opposed. Header bay **604** houses header assembly **502**. Likewise, header bay **606** houses header assembly **504.**

Header bay **604** includes rectangular receiving window **618,** and electrical pass-through holes **614**. The receiving window and the electrical pass-through holes allow for connections between header assembly **502** and the internal components of the IPG.

Likewise, header bay **606** includes rectangular receiving window **620** and electrical pass-through holes **616** for connection of the components of header assembly **504** with the internal components of the IPG.

Header assembly **502** includes integrally formed positioning block **610**. Positioning block **610** fits within receiving window **618** and optically aligns the header bay with the optical components in the case. Header assembly **504** includes integrally formed positioning block **612**. Positioning block **612** fits within receiving window **620** and optically aligns the header bay with the optical components in the case.

Composite case **402** including both headers forms an elliptical surface of revolution as described in U.S. Publication No. 2021/0001114 to Wolf, incorporated herein by reference.

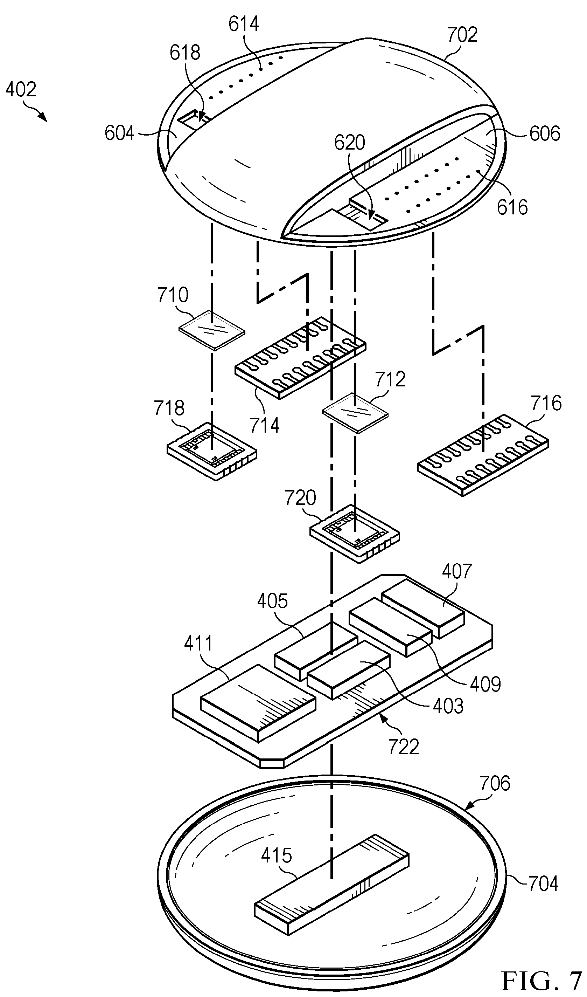

Referring to Figure 7, composite case **402** will be further described.

Composite case **402** includes top section **702** joined to bottom section **704**. Top section **702** is preferably a hollow shell manufactured from a titanium alloy. The titanium alloy is relatively easily machined and can hold tolerances sufficient to allow proper alignment of headers and optical components, as will be further described. Bottom section **704** is preferably a hollow shell comprised of a ceramic material. The ceramic material facilitates radio communication between the communication circuits and inductive coupling between the inductive coils. The combination of a ceramic material with a metallic material for the case is also important because the heat generated from the IPG is directed toward the metallic section of the case. The metallic section of the case may be positioned toward the dermis during surgery, thus allowing the IPG to be positioned for superior heat dispersion while in use.

Optical window **710** is positioned adjacent top section **702** and centered beneath receiving window **618**. Likewise, optical window **712** is positioned adjacent top section **702** and centered beneath receiving window **620**. Both optical windows are preferably welded and sealed to the underside of the top section.

Die stack **718** is functionally positioned beneath optical window **710** and adjacent optical signal processor **405,** as will be further described. Die stack **720** is functionally positioned beneath optical window **712** and adjacent optical signal processor **403,** as will be further described.

Connector card **714** is positioned adjacent top section **702** and directly beneath electrical pass-through holes **614**. Connector card **716** is positioned adjacent top section **702** directly beneath electrical pass-through holes **616**. Connector card **714** and connector card **716** are electrically connected to contacts in the headers and main processor **407,** and allow for transmission of stimulation current to the leads, as will be further described.

Composite case **402** houses processor card **722**. Processor card **722** structurally and electrically connects main processor **407** to optical signal processors **405** and **403,** signal generator **409** and communications circuit **411**. In a preferred embodiment, main processor **407** is Part No. MSP430, available from Texas Instruments of Dallas, Texas. Signal generator **409** is available under the tradename Saturn, available from Cactus Semiconductor. Communications circuit **411** is preferably Part No. ZL70103, available from Microsemi Corporation of Aliso Viejo, California. Optical signal processors **403** and **405** are both preferably Part No. ADPD4100, available from Analog Devices of Wilmington, Massachusetts.

Composite case **402** further houses battery **415** which is electrically connected to processor card **722**. The operational components of the IPG are preferably positioned adjacent top section **702** and held in place by an epoxy encapsulation. After encapsulation, bottom section **704** is hermetically sealed to top section **702** by weldment **706**.

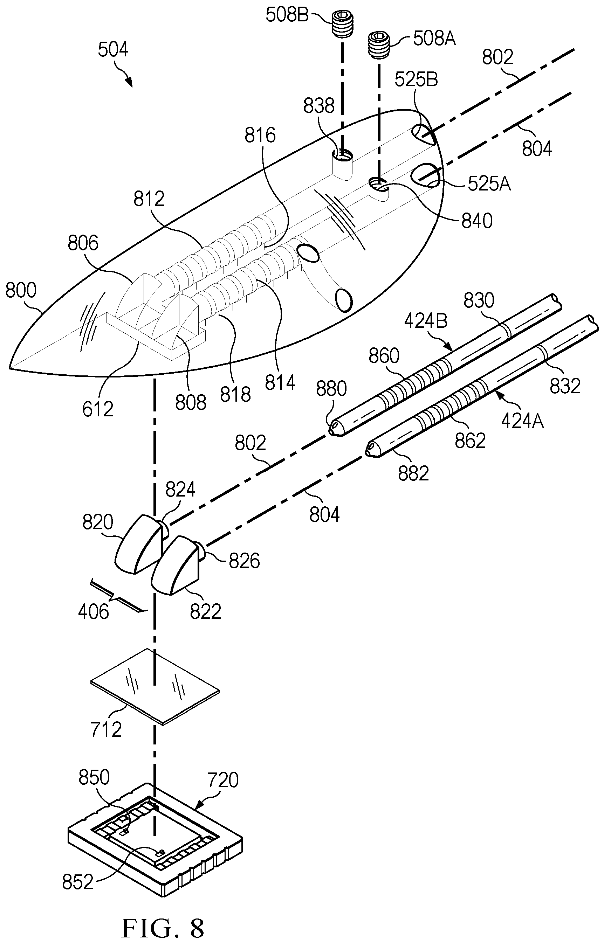

Referring then to Figure 8, header assembly **504** will be further described. It should be understood that header assembly **502** is structurally and functionally identical to header assembly **504,** with the exception of the components being in reversed positions. Only header assembly **504** will be described in detail here as an example.

Header assembly **504** includes header body **800**. Header body **800** preferably is manufactured from a transparent epoxy resin, or acrylic plastic, cast and machined to tolerance. Header body **800** is formed in a semi-ellipsoidal shape, sufficient to fit seamlessly within header bay **606** and comport with the surface of revolution formed by top section **702** and bottom section **704**.

Header body **800** includes lead retainer holes **525A** and **525B**. Lead retainer hole **525A** includes central optical axis **804**. Lead retainer hole **525B** includes central optical axis **802**. Optical axis **802** and optical axis **804,** preferably, are generally parallel.

Lead retainer hole **525A** includes metallic toroidal contacts **814**. In a preferred embodiment, eight toroidal contacts are included, each individually addressable by the main processor. Toroidal contacts **814** are each connected to a lead wire **818**. Lead wires **818** are positioned to intersect electrical pass-through holes **616** and be connected to the connector card which further connects them to the main processor. Lead retainer hole **525A** terminates in cavity **808,** adjacent positioning block **612,** as will be further described.

Lead retainer hole **525B** includes metallic toroidal contacts **812**. In a preferred embodiment, eight toroidal contacts are included, each individually addressable by the main processor. Toroidal contacts **812** are each connected to a lead wire **816**. Lead wires **816** are positioned to intersect electrical pass-through holes **616** and be connected to the connector card which further connects them to the main processor. Lead retainer hole **525B** terminates in cavity **806** adjacent positioning block **612,** as will be further described.

Lead retainer hole **525A** includes threaded perpendicular hole **840**. When lead **424A** is positioned within lead retainer hole **525A,** set screw **508A** is positioned in threaded perpendicular hole **840** directly adjacent anchor ring **832** and secures lead **424A** in lead retainer hole **525A.**

Likewise, lead retainer hole **525B** includes threaded perpendicular hole **838**. Set screw **508B** is positioned within threaded perpendicular hole **838** directly adjacent to lead anchor ring **830**. Set screw **508B** is advanced to contact lead anchor ring **830** and holds lead **424B** in lead retainer hole **525B**.

Optical folding assembly **406** further comprises parabolic redirector **820** and parabolic redirector **822**. Parabolic redirector **820** includes integral lens **824**. Parabolic redirector **822** includes integral lens **826**. Parabolic redirector **820** and parabolic redirector **822** are rigidly secured in cavity **806** and cavity **808,** respectively, in contact with and adjacent to optical window **712,** as will be further described. In a preferred embodiment, the parabolic redirectors are cast in place in the header. Integral lens **824** is optically aligned with optical axis **802**. Integral lens **826** is optically aligned with optical axis **804**.

Die stack **720** is positioned directly below and in contact with optical window **712**. Die stack **720** includes vertical cavity surface emitting laser ("VCSEL") **850** and VCSEL **852,** as will be further described. In each case, the VCSELs are capable of emitting light in an intrinsic wavelength range, but preferably in the range of about 400 - 810 nanometers or from blue (about 400 nanometers to about 500 nanometers), to green (about 520 nanometers to about 532 nanometers) to near IR (about 700 nanometers to about 810 nanometers). In a preferred embodiment, each VCSEL is Part No. V00146, available from Vixar, Inc. of Plymouth, Minnesota. Each laser produces about 10 milliwatts in the range of near IR and about 5 milliwatts in the blue range. Other lasers may be utilized in the wavelength range of about 400 - 580 nanometers (blue, aqua, green and yellow) as well as other visible ranges. VCSEL **850** is positioned to emit light vertically toward the central vertical optical axis of parabolic redirector **820**. Likewise, VCSEL **852** is positioned to emit light vertically toward the central vertical optical axis of parabolic redirector **822**.

Toroidal contacts **812** are designed to engage and electrically contact rings **860** of lead **424B,** as will be further described. Toroidal contacts **814** are designed to engage and electrically contact rings **862** of lead **424A,** as will be further described.

Lead **424B** is positioned to align with optical axis **802** by lead retainer hole **525B**. Lead **424A** is positioned to align with optical axis **804** by lead retainer hole **525A**.

Lead **424B** terminates in collet **880,** as will be further described. Lead **424A** terminates in collet **882,** as will be further described. When assembled, lead **424B** and collet **880** are held adjacent to integral lens **824** by set screw **508B**. Lead **424A** and collet **882** are held adjacent integral lens **826** by set screw **508A**. An index matching gel is provided to minimize Fresnel reflections between the lenses and the leads.

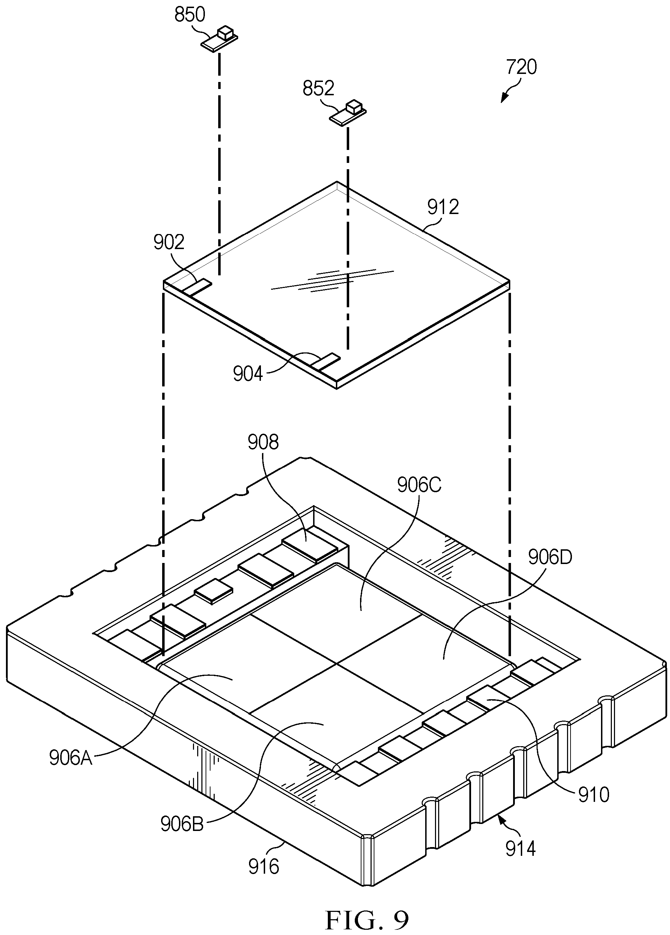

Referring in to Figure 9, die stack **720** will be further described. It should be understood that die stack **718** and die stack **720** are functionally and structurally identical. Only die stack **720** will be described in detail.

Die stack **720** includes photodiode package **914**. In a preferred embodiment, photodiode package **914** is Part No. S5980-09(ESI), available from Hamamatsu Photonics K.K. of Shizuoka, Japan.

The preferred photodiode package has four photodiodes. In one embodiment, all four photodiodes are used as photoreceivers. In another embodiment, two photodiodes **906A** and **906B** may be used as photoreceivers, one for each lead, and two photodiodes **906C** and **906D,** are used as monitor photodiodes one for each laser. The two monitor photodiodes act as output monitors to normalize the laser emission and correct for source power variations over time, as will be further described.

Photodiodes **906A, 906B, 906C** and **906D** are recessed and fixed in housing **916**. Housing **916** is preferably a ceramic composite. Housing **916** is held in position in the case by epoxy encapsulation and rigidly fixes the position of the photodiodes. Each of the photodiodes typically provides a sensitivity of about 0.72 A/W. Photodiodes **906A, 906B, 906C** and **906D** are bounded by electrical contacts **908** and **910**. These contacts are operatively connected to the appropriate optical signal processor by flexible cables (not shown). Cover plate **912** is positioned adjacent to and in contact with the four photodiodes. In a preferred embodiment, cover plate **912** is formed of a crystal glass polished to have optically parallel opposing faces. Cover plate **912** includes gold trace **902** and gold trace **904**. The gold traces are preferably deposited on the glass surface opposite the photodiodes using photolithography or vapor deposition. VCSEL **850** is rigidly fixed to cover plate **912** adjacent to and in electrical contact with gold trace **902**. VCSEL **852** is rigidly fixed to optical window **712** adjacent to and in electrical contact with gold trace **904**. The gold traces provide power to the VCSELs and allow the main processor to select which wavelength laser to activate, as will be further described. Gold trace **902**, gold trace **904,** and contacts **908** and **910** are electrically connected to the appropriate optical signal processor and main processor **407** by flexible cables (not shown).

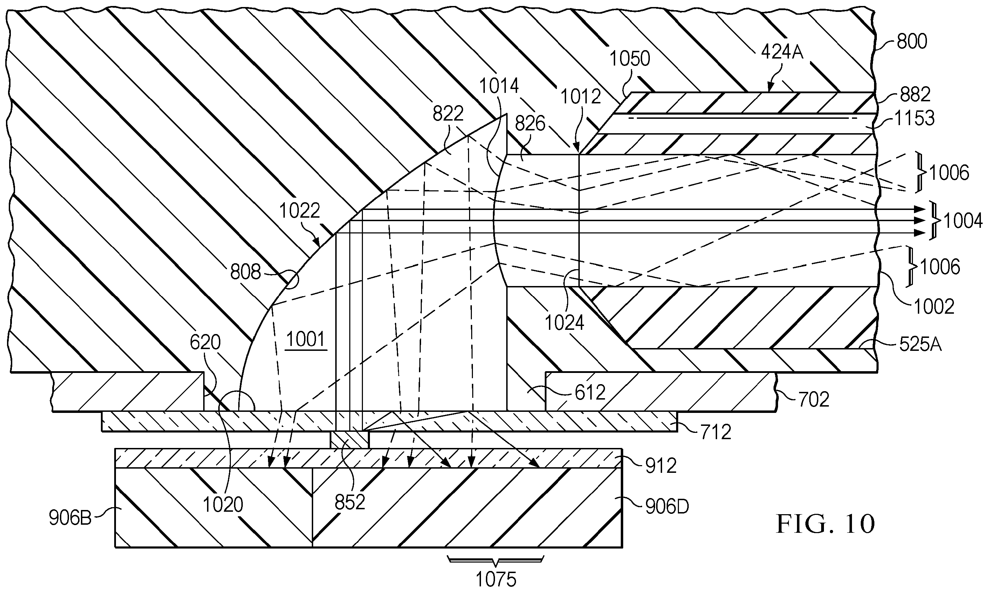

Referring then to Figure 10, a partial cross section view of IPG **401,** in use, will be further described. It should be understood that each parabolic redirector, die stack and mating components in the header and lead are structurally and functionally identical, hence only one set will be described here as an example.

Parabolic redirector **822** is further comprised of parabolic body **1001** and integral lens **826**. Parabolic body **1001** and integral lens **826** are preferably integrally formed from a crystal glass having an index of refraction of between about 1.46 and 1.68, similar to that of silicone resin.

Parabolic body **1001** includes parabolic surface **1022**. Parabolic surface **1022** is preferably a paraboloid having a curvature designed to produce one focal point at the VCSEL and another focal point at the face of and aligned to the optical axis of the fiber.

Parabolic surface **1022** includes an exterior reflective coating, preferably vapor deposited silver. In other embodiments, parabolic surface **1022** is coated with a titanium dioxide compound. Preferably, parabolic surface **1022** is also polished.

Parabolic body **1001** further includes interface surface **1020** adjacent optical window **712**. Interface surface **1020** is flat to within an acceptable optical tolerance. Preferably, an index matching material, such as an epoxy is resident adjacent the surface and the optical window to provide low reflection loss and part stability. In another preferred embodiment, interface surface **1020** may be positioned adjacent optical window **712** with an index matching gel and fixed in place with a suitable epoxy adhesive.

Parabolic redirector **822** is further comprised of integral lens **826**. Integral lens **826** is a collimating lens and includes convex lens surface **1014** which is directed inward toward parabolic surface **1022**. In one preferred embodiment, convex lens surface **1014** is fixed to parabolic body **1001** by a suitable index matching epoxy. In another preferred embodiment, the parabolic body and the integral lens are integrally formed. In this case, the convex lens surface is created by an appropriate density change between integral lens **826** and parabolic body **1001**. Integral lens **826** includes interface surface **1024** adjacent fiber 1002. Interface surface **1024** is preferably ground flat within appropriate optical tolerances.

Header body **800** includes frustroconical receiver surface **1050** at the proximal end of lead retainer hole **525A**. Interface surface **1024** is positioned parallel to and against fiber **1002** of lead **424A** at optical interface **1012** and held in place by the interference between collet **882** and frustroconical receiver surface **1050** of lead retainer hole **525A**.

Positioning block **612** forms an extension of the header body and serves to position the parabolic redirector. The position block is positioned within receiving window **620** of top section **702**. The interface between receiving window **620** and positioning block **612** fixes the vertical optical axis of the parabolic redirector above VCSEL **852** and in position to reflect light from parabolic surface **1022** to optical axis **804** of fiber **1002** of lead **424A**.

In use, VCSEL **852** produces laser light pulses **1004** which proceed vertically upward toward parabolic surface **1022**. The parabolic surface reflects the light about 90 degrees from vertically upward to horizontal and aligns the light through the integral lens and into fiber **1002**, along optical axis **804**, exiting the fiber at its distal end toward the spinal cord. In all cases, the pulses from the VCSELs will be specularly reflected from the spinal cord, given its high multispectral albedo. However, blue, aqua, green or yellow pulses will be preferentially absorbed by surrounding tissues and by any hemoglobin, if present, while in the case of IR, the pulses will be preferentially reflected from these tissues. Upon reflecting from the spinal cord, receive rays **1006** are collected and retransmitted through fiber **1002** back to the parabolic redirector. Receive rays **1006** are not well aligned along the fiber and so impact integral lens **826** at various angles. Receive rays **1006** are expanded by convex lens surface **1014** where they are incident upon parabolic surface **1022**, where they are collected and directed about 90 degrees from horizontal to vertically downward, toward optical window **712**. Receive rays **1006** pass through optical window **712** and are incident on cover plate **912** where they are directed toward photodiodes **906B** and **906D**.

When VCSEL **852** is in operation, some small percentage of light pulses **1004** are back reflected from optical window **712**. These back reflected pulses **1075** are incident immediately on cover plate **912**, where they are transmitted to photodiode **906D**. The back reflected pulses are converted to signals by the photodiode that are used by the main processor to normalize the power output of the VCSEL and correct for source power variation, as will be further described.

Photodiodes **906B** and **906D** convert receive rays **1006** into electrical signals which are communicated to optical signal processor **405** for further processing, as will be further described.

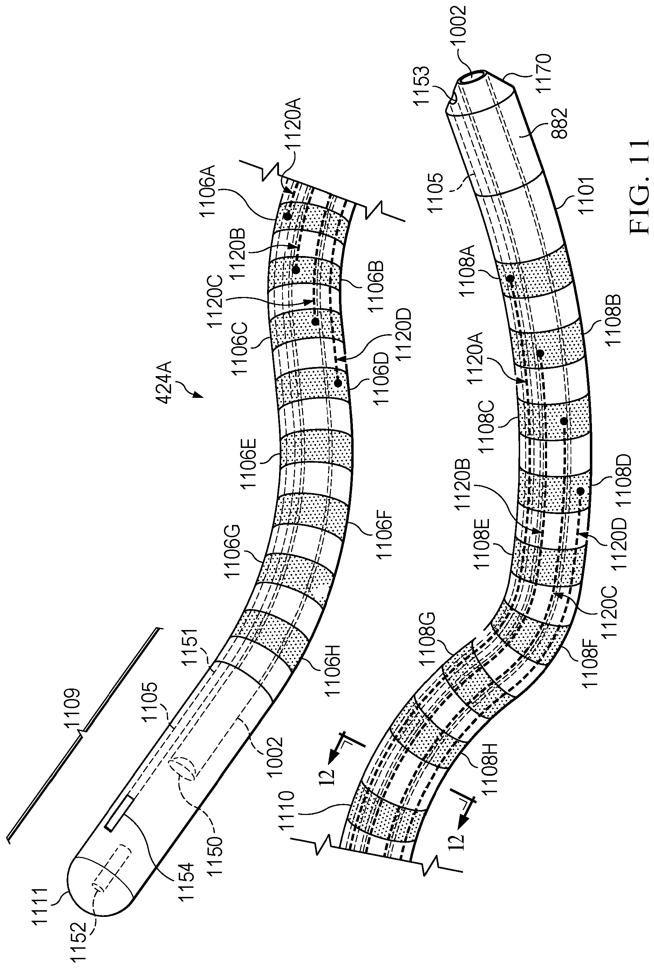

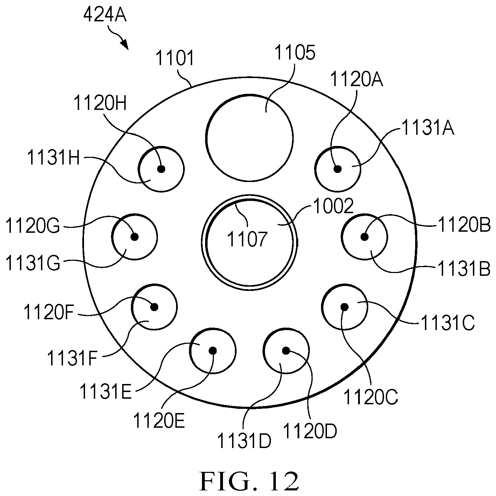

Referring then to Figures 11 and 12, lead **424A** will be further described. Lead **424A**, lead **424B**, lead **422A** and lead **422B** are identical in structure and function. Only one will be described here, by way of example.

Lead **424A** further comprises lead body **1101**. Lead body **1101** is generally a flexible cylindrical extrusion distally terminated by transmission tip **1109** and proximally terminated by collet **882**. In a preferred embodiment, the lead body is comprised of a flexible polymer, such as Pellethane 55D or similar biocompatible material. The lead body is preferably a multi-lumen extrusion having embedded and integrally formed components, as will be further described.

Transmission tip **1109** is an optically transparent cylinder fused to the distal terminus of the lead body. In a preferred embodiment, the transmission tip is a suitable optically transparent material such as a thermoplastic polyurethane. Transmission tip **1109** is terminated by semi-spherical cap **1111**. In a preferred embodiment, semi-spherical cap **1111** and transmission tip **1109** are integrally formed. Transmission tip **1109** further includes embedded radiopaque marker **1152**. Radiopaque marker **1152** is preferably titanium cylinder axially embedded adjacent semi-spherical cap **1111**.

Fiber **1002** is positioned along the central optical axis of lead **424A** and extends from collet **882** to concave lexicon **1150**. The transmission tip is fused to fiber **1002**. Fiber **1002** includes concave lexicon **1150** at its distal end. In a preferred embodiment, concave lexicon **1150** includes internally reflective coating such as titanium dioxide. Transmission tip **1109** further includes stylet channel terminus **1151**. In a preferred embodiment, stylet channel terminus **1151** is a cylindrical opening. Stylet stop **1154** is positioned at the distal end of stylet channel terminus **1151**. Stylet stop **1154** is preferably a titanium cylinder.

Stylet channel **1105** is coaxial with and extends from stylet channel terminus **1151** to stylet channel opening **1153,** in collet **882.** The stylet channel is a cylindrical cavity which runs parallel with the optical fiber and serves the purpose of housing a guide stylet for use during placement of the lead during surgery. In preferred embodiment, stylet channel **1105** is lined with a polytetrafluoroethylene (PTFE) lining **1107,** which extends the length of the lead body. The low surface friction afforded by the lining facilitates insertion of the stylet during surgery.

Lead body **1101** further supports metallic anchor **1110** positioned at its proximal end. The metallic anchor is generally cylindrical and is permanently affixed to the exterior of the lead body.

Adjacent metallic anchor **1110,** are eight cylindrical proximal metallic contacts, **1108A, 1108B, 1108C, 1108D, 1108E, 1108F, 1108G,** and **1108H** are fixed to the exterior of the lead body at even axial distances and are positioned to electrically contact the toroidal contacts in the header assembly.

Likewise, eight cylindrical metallic electrodes **1106A, 1106B, 1106C, 1106D, 1106E, 1106F, 1106G,** and **1106H** are fixed to the distal end of the lead body. The metallic electrodes are each permanently fixed to the exterior surface of the lead body, at equal axial distances.

The lead body further comprises eight radially oriented lumens, **1131A, 1131B, 1131C, 1131D, 1131E, 1131F, 1131G,** and **1131H.** Conductors **1120A, 1120B, 1120C, 1120D, 1120E, 1120F, 1120G** and **1120H** are integrally formed in the lumens and extend from their respective proximal contacts to their respective distal electrodes. In a preferred embodiment, the conductors are comprised of MP35N, or another conductive material similarly resistant to corrosion. Each of the conductors to connect exactly one proximal contact with exactly one paired metallic electrode.

In a preferred embodiment, fiber **1002** and the conductors are integrally formed into the lead body during manufacture.

In a preferred embodiment, collet **882** is formed from a suitable ceramic or crystal sapphire material. Collet **882** includes frustoconical surface **1170** at its proximal end. The frustoconical surface mates with an identical frustoconical receiver surface **1050** in header body **800** and aids in positioning fiber **1002** against optical interface **1012,** and in radially compressing the fiber to aid in optical alignment with the parabolic redirector.

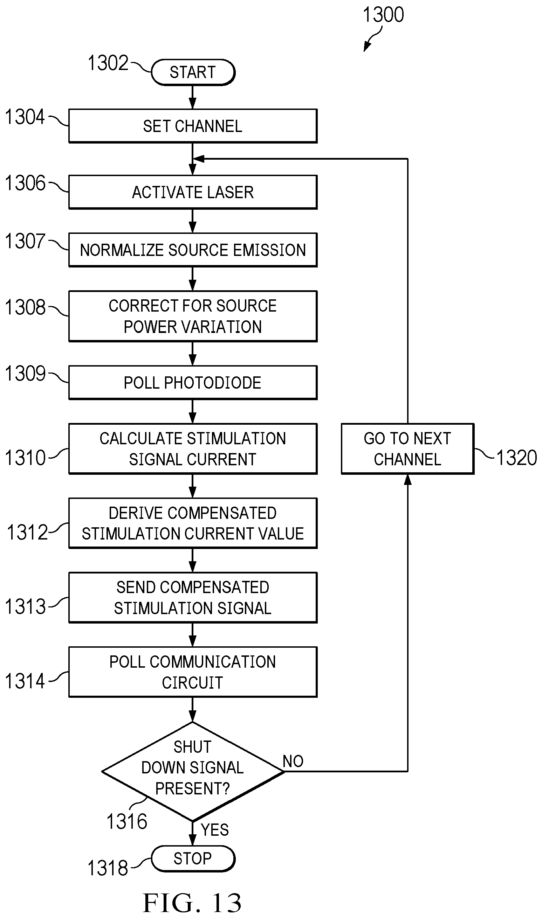

Referring to Figure 13, method of IPG operation **1300** will be further described. In preferred embodiment, the method is carried out by programming instructions, which are resident in onboard memory of main processor **407.**

At step **1302,** the method begins.

At step **1304,** the main processor sets an initial channel for operation. In a preferred embodiment, an initial channel includes one of the group of four leads **422A, 422B, 424A** and **424B.** In other preferred embodiments, the initial channel includes one of the group of only two or three of the leads.

In each case, the main processor addresses only eight electrodes on one lead at any one time. Each of the eight electrodes on the lead selected may be individually addressed by the main processor with a different current level for the stimulation signal.

At step **1306,** main processor **407** activates the VCSEL for the specified channel. Preferably, the VCSEL produces a light pulse in the IR wavelength range. A small percentage of the light pulse is immediately back reflected by the optical window. The majority of the light pulse is sent to the base of the parabolic redirector where it passes to the parabolic surface which turns the light about 90 degrees from vertical to the horizontal and focuses it along the optical axis of the optical fiber for the chosen lead where it traverses the lead and exits from the concave lexicon through the transmission tip. The transmitted ray then is incident on the spinal cord, hemoglobin and other surrounding tissues, where it is reflected and received by the fiber at the concave lexicon. The received ray is transmitted down the fiber returning to the parabolic redirector where it is turned about 90 degrees, from the horizontal to vertically downward, and focused on the die stack.

At step **1307,** main processor **407** uses the back reflected pulse to normalize the source emission, as will be further described.

At step **1308,** the main processor uses the back reflected pulse to correct for power variation over time, will be further described.

At step **1309,** the main processor polls the photodiode to obtain a feedback signal.

At step **1310,** the main processor calculates a stimulation signal based on the signal from the photodiode. The stimulation signal is preferably generated according to a table, accurately disclosed in U.S. Patent No. 9,550,063 to Wolf II, incorporated herein by reference. Of course, other stimulation routines may be used.

At step **1312,** the main processor modifies the stimulation signal to compensate for the reduced optical feedback, according to the reflection compensation value, as will be further described.

At step **1313,** the main processor activates the signal generator to send the compensated stimulation signal to the toroidal contacts for the lead for the chosen channel. The compensated stimulation signal is transmitted to the electrodes, which creates an electric field adjacent the target neurons at the spinal cord.

At step **1314,** the main processor polls the communication circuit for a shutdown signal.

At step **1316,** the main processor determines whether or not a shutdown signal is present. If not, the main processor moves to step **1320.** If so, the main processor moves to step **1318.**

At step **1320,** the main processor advances to the next lead channel in the group of leads and returns to step **1306.**

At step **1318,** the main processor shuts down the routine and returns to a holding state.

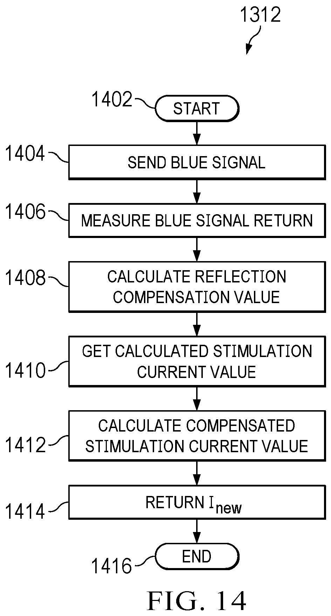

Referring then to Figure 14, a preferred method of deriving a compensated stimulation current value of step **1312,** will be further described.

At step **1402,** the method begins.

At step **1404,** the main processor activates the VCSEL to generate a second light pulse. Preferably, the VCSEL produces a light pulse in the blue wavelength range. However, other wavelengths may be used to long as they are a different wavelength than the first pulse. The blue laser signal proceeds through the parabolic redirector where it enters the fiber and is transmitted to the concave lexicon at the transmission tip. However, the blue light is preferentially absorbed by the surrounding tissues and hemoglobin. Most or all of the blue signal is absorbed by these tissues. As a result, the blue signal reflected is due primarily to Fresnel reflections in the optical system. Hence, the reflected blue signal represents optical system signal loss between the blue signal origination at the VCSEL and the blue signal reception at the photoreceiver. By monitoring the blue light reflections periodically, the optical system signal loss and increase in system noise over time can be recognized.

The optical system signal loss and noise are the same for each light signal regardless of wavelength. Hence, the optical signal system loss for the IR signal pulse will be the same as the optical signal system loss for the blue signal pulse. The preferred electro-optical controller generally increases stimulation current when the optical signal is decreased in intensity, based on the assumption that the spinal cord is farther away from the electrodes. So, as the optical losses in the system increase, the controller gradually increases the stimulation current. By decreasing the stimulation current by the same percentage that the optical losses increase, the optical degradation and Fresnel loss in the system can be compensated for, thereby providing the correct stimulation signal.

At step **1406,** the return blue laser signal is measured by the photodiode which returns a current value to the main processor.

At step **1408,** the main processor preferably calculates the percentage signal loss in the blue optical signal, known as the reflection compensation value, according to the following equation.

$$RCV = \frac{I_t - I_r}{I_t} \times 100$$

Where:

RCV = percentage optical signal loss;
$I_t$ = the transmitted optical signal current to the VCSEL; and,
$I_r$ = the received optical signal current from the photodiode.

At step **1410,** the stimulation current value,

"$I_{stim}$", is retrieved. $I_{stim}$ is the current value calculated by the processor according to the correlation table between the return optical signal and the stimulation current, as previously described.

At step **1412,** the stimulation current correction value, "$I_{new}$", is preferably calculated according to the following equations.

$$RCV \times I_{stim} = I_{new}$$

Where:

$I_{stim}$ = calculated stimulation current;
$RCV$ = percentage optical signal loss; and
$I_{new}=$

At step **1414,** the main processor returns the $I_{new}$ value.

At step **1416,** the method concludes.

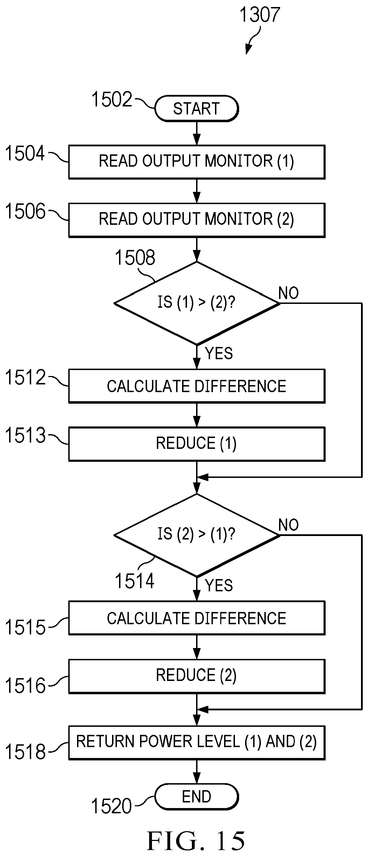

Referring then to Figure 15, a preferred method of normalizing source emission of step **1307** will be further described. The method is the same for each die stack.

At step **1502,** the method begins.

At step **1504,** main processor **407** retrieves from memory the output of monitor photodiode one. In this example, photodiode one is photodiode **906C** adjacent VCSEL **850.**

At step **1506,** the main processor reads the output value of monitor photodiode two. In this example, photodiode two is photodiode **906D** adjacent VCSEL **852.**

At step **1508,** the main processor compares the output of monitor photodiode one and the output of monitor photodiode two. If the output of photodiode one is greater than the output of photodiode two, then the method moves to step **1512.** If not, the method moves to step **1514.**

At step **1512,** the difference between the output of photodiode one and the output of photodiode two is calculated by subtracting the output of photodiode two from the output of photodiode one.

At step **1513,** the drive current to photodiode one is reduced by the difference.

At step **1514,** the output of photodiode two is compared to the output of photodiode one. If the output of photodiode two is greater than the output of photodiode one, then the method moves to step **1515.** If not, the method moves to step **1518.**

At step **1515,** the difference between the output of photodiode two and the output of photodiode one is calculated by subtracting the output of photodiode one from the output of photodiode two.

At step **1516,** the drive current to photodiode two is reduced by the difference.

At step **1518,** the method returns the normalized power levels for photodiode one and photodiode two.

At step **1520,** the method ends.

In a preferred embodiment, the differences are calculated as percentages which can be directly applied to the photodiode current to normalize the source of emission.

In one preferred embodiment, method **1307** is applied between the two VCSELs in one photodiode stack, such as VCSEL **850** and VCSEL **852.** However, in other embodiments, normalization may take place over all four VCSELs in the system, so that each produces the same level of source emission.

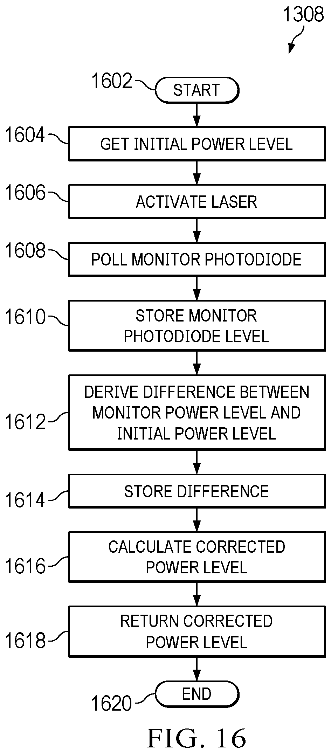

Referring then to Figure 16, a preferred method of correcting for source power variation of step **1308** will be further described.

At step **1602,** the method begins. Ideally, the method takes place only periodically, such as once a week, so as to conserve system power usage.

At step **1604,** the main processor retrieves the initial power output level of the VCSEL from memory. Preferably, the initial power output level is measured by measuring the current from the monitor photodiode adjacent the VCSEL when the VCSEL is initially activated during system startup.

At step **1606,** the main processor activates the chosen VCSEL.

At step **1608,** the main processor polls the monitor photodiode for the chosen VCSEL.

At step **1610,** the main processor stores the monitor photodiode power level.

At step **1612,** the main processor derives a difference between the monitor photodiode power level and the initial monitor photodiode power level. According to the following equation:

$$PL_I - PL_m = \Delta$$

Where:

$\Delta$ = the difference;
$PL_I$ = the initial power level; and
$PL_m$ = the measured power level.

At step **1614,** the difference is stored as a percentage according to the following equation:

$$PL_{DIFF} = \frac{PL_I - PL_m}{100}$$

Where:

$PL_{DIFF}$ = the percentage power level difference;
$PL_I$ = the initial power level; and
$PL_m$ = the measured power level.

At step **1616,** the corrected power level is calculated according to the following equation:

$$I_1 \times PL_{DIFF} = I_2$$

Where:

$I_1$ = the initial current level;
$PL_{DIFF}$ = the percentage difference; and
$I_2$ = the new power level.

At step **1618,** the main processor returns the new power level.

At step **1620,** the method concludes.

**Claims**

1. An implantable pulse generator system comprising:

   a case (401);
   a lead retainer hole (523A, 523B, 525A, 525B), longitudinally positioned in the case, having an optical axis;
   a parabolic redirector (820, 822), having a first interface surface perpendicular to a second interface surface connected by a parabolic surface, focused on the optical axis;
   a die stack (718, 720), adjacent the parabolic redirector, perpendicular to the optical axis and parallel to the second interface surface;
   a laser (850, 852), fixed on the die stack, directed vertically toward to the second interface surface;
   a photo receiver (906A, 906B, 906C, 906D), positioned around the laser and parallel to the second interface surface; and
   a processor (407), having a memory, operatively connected to the laser and the photo receiver.

2. The implantable pulse generator system of claim 1, wherein the laser is a VCSEL, or

   wherein the parabolic redirector includes a collimating lens (826) centered on the optical axis, or
   wherein the laser is fixed on the die stack by an optical window, preferably wherein the laser is electrically connected to the processor by a metallic trace, fixed on the optical window, or
   wherein the case further comprises:

      a metallic first section forming a header bay;
      a header, containing the lead retainer hole, fixed in the header bay; and
      a ceramic second section, hermetically sealed to the metallic first section.

3. The implantable pulse generator system of claim 1, further comprising:

a set of toroidal electrical contacts (812, 814), fixed in the lead retainer hole, axially aligned with the optical axis.

4. The implantable pulse generator system of claim 3, further comprising:

a flexible lead (422A, 422B, 424A, 424B), fixed in the lead retainer hole;
a centrally disposed optical fiber (1002), integrally formed in the flexible lead, coaxial with the optical axis;
a set of cylindrical contacts (1108A, 1108B, 1108C, 1108D, 1108E, 1108F, 1108G, 1108H), fixed on an exterior surface of the flexible lead, electrically connected to the set of toroidal electrical contacts; and
a set of cylindrical electrodes (1106A, 1106B, 1106C, 1106D, 1106E, 1106F, 1106G, 1106H), fixed on the exterior surface of the flexible lead, electrically connected to the set of cylindrical contacts.

5. The implantable pulse generator system of claim 4, wherein the flexible lead further comprises:
a longitudinal stylet lumen (1131A, 1131B, 1131C, 1131D, 1131E, 1131F, 1131G, 1131H), radially disposed adjacent and parallel to the centrally disposed optical fiber, preferably wherein the longitudinal stylet lumen terminates distally in a stylet stop (1154), or the flexible lead further comprises:
a transparent optical transmission tip (1109), integrally formed with the centrally disposed optical fiber, preferably wherein the transparent optical transmission tip further comprises a centrally disposed radiopaque marker (1152).

6. The implantable pulse generator system of claim 4, further comprising:
a set of instructions, resident in the memory, that when executed cause the implantable pulse generator system to:

generate a first transmit ray, at a first wavelength, from the laser;
send the first transmit ray through the parabolic redirector down the centrally disposed optical fiber;
receive a first receive ray, from the centrally disposed optical fiber, through the parabolic redirector, incident on the photo receiver;
generate a variation variable from the first receive ray;
create a modulated stimulation signal based on the variation variable; and
send the modulated stimulation signal to the set of cylindrical electrodes, preferably wherein the set of instructions comprises further instructions, resident in the memory, that when executed cause the implantable pulse generator system to:

generate a second transmit ray, of a second wavelength, from the laser;
send the second transmit ray through the parabolic redirector down the centrally disposed optical fiber;
receive a second receive ray, from the centrally disposed optical fiber, through the parabolic redirector incident on the photo receiver;
generate a compensation value from the second receive ray; and
alter the modulated stimulation signal based on the compensation value.

7. An implantable pulse generator system comprising:

a case (401);
a first header bay (604), formed in the case;
a second header bay (606), diametrically disposed to the first header bay, formed in the case;
a first header assembly (502), fixed in the first header bay, having a first lead retainer channel (523A) and a second lead retainer channel (523B);
a second header assembly (504), fixed in the second header bay, having a third lead retainer channel (525A) and a fourth lead retainer channel (525B);
a first electro-optical lead (422A), having a first optical axis, positioned in the first lead retainer channel;
a second electro-optical lead (422B), having a second optical axis, positioned in the second lead retainer channel;
a third electro-optical lead (424A), having a third optical axis, positioned in the third lead retainer channel;
a fourth electro-optical lead (424B), having a fourth optical axis, positioned in the fourth lead retainer channel;
a first parabolic redirector, centered on the first optical axis, optically coupled to the first electro-optical lead;
a second parabolic redirector, centered on the second optical axis, optically coupled to the second electro-optical lead;
a third parabolic redirector (822), centered on the third optical axis, optically coupled to the third electro-optical lead;
a fourth parabolic redirector (820), centered on the fourth optical axis, optically coupled to the fourth electro-optical lead;
a first die stack (718), having a first perpendicularly oriented laser, surrounded by a first photodiode, optically coupled to the first parabolic redirector;

the first die stack, having a second perpendicularly oriented laser, surrounded by a second photodiode, optically coupled to the second parabolic redirector;

a second die stack (720), having a third perpendicularly oriented laser (850), surrounded by a third photodiode (906A), optically coupled to the third parabolic redirector; and

the second die stack, having a fourth perpendicularly oriented laser (852), surrounded by a fourth photodiode (906B), optically coupled to the fourth parabolic redirector.

**8.** The implantable pulse generator system of claim 7, wherein the first electro-optical lead further comprises:

an optical fiber (1002), positioned coaxially with the first optical axis;

a set of electrical contacts (1108A, 1108B, 1108C, 1108D, 1108E, 1108F, 1108G, 1108H), fixed on a proximal surface of the first electro-optical lead; and

a set of electrodes (1106A, 1106B, 1106C, 1106D, 1106E, 1106F, 1106G, 1106H), fixed on a distal surface of the first electro-optical lead, electrically connected to the set of electrical contacts.

**9.** The implantable pulse generator system of claim 8, wherein the first header bay further comprises:

a set of fixed contacts (812), rigidly positioned in the first lead retainer channel, and operatively connected to an electro-optical signal generator;

wherein the electro-optical signal generator is programmed to:

send a first transmit ray, of a first wavelength, from the first perpendicularly oriented laser into the first parabolic redirector and the optical fiber;

receive a first return signal from the first photodiode, based on a first receive ray;

generate a stimulation signal based on the first return signal; and

send the stimulation signal to the set of fixed contacts, for transmission to the set of electrodes.

**10.** The implantable pulse generator system of claim 9, wherein the electro-optical signal generator is further programmed to:

send a second transmit ray, of a second wavelength, from the first perpendicularly oriented laser into the first parabolic redirector and the first electro-optical lead;

receive a second receive ray at the first photodiode;

generate a compensation value from the second receive ray; and modify the stimulation signal based on the compensation value, preferably , wherein:

the first wavelength is between about 700 nanometer and about 800 nanometers; and the second wavelength is between about 400 nanometers and about 500 nanometers or between about 520 nanometers and about 532 nanometers.

**11.** The implantable pulse generator system of claim 9, wherein:

the first die stack further comprises a second photodiode;

wherein the electro-optical signal generator is further programmed to:

normalize a first supply current to the first photodiode and a second supply current to the second photodiode, preferably wherein the step of normalizing further comprises:

reducing the first supply current if the first supply current is greater than the second supply current; and

reducing the second supply current if the second supply current is greater than the first supply current, or

the electro-optical signal generator is further programmed to:

correct for a time based variation in the first supply current, preferably wherein the step of correcting further comprises:

deriving a difference between an initial supply current to the first photodiode and the first supply current.

**12.** The implantable pulse generator system of claim 7, wherein the first parabolic redirector further comprises:

a collimating lens centered on the first optical axis; and

a parabolic surface, for reflecting a transmit ray toward the first electro-optical lead and a receive ray toward the first photodiode, preferably wherein the parabolic surface includes a reflective coating, or

further comprising:

a transparent cover plate, between the first perpendicularly oriented laser and the first die stack, positioning the first perpendicularly oriented laser adjacent the first parabolic redirector, preferably further comprising:

a window, sealed to the case, between the first perpendicularly oriented laser and the first parabolic redirector.

## Patentansprüche

1. Implantierbares Impulsgeneratorsystem, umfassend:

   ein Gehäuse (401);
   ein Leitungshalteloch (523A, 523B, 525A, 525B), in Längsrichtung positioniert in dem Gehäuse, aufweisend eine optische Achse;
   einen parabolischen Umlenker (820, 822), aufweisend eine erste Schnittstellenoberfläche senkrecht zu einer zweiten Schnittstellenoberfläche, verbunden durch eine parabolische Oberfläche, fokussiert auf die optische Achse;
   einen Chipstapel (718, 720), benachbart zu dem parabolischen Umlenker, senkrecht zu der optischen Achse und parallel zu der zweiten Schnittstellenoberfläche;
   einen Laser (850, 852), befestigt auf dem Chipstapel, vertikal zu der zweiten Schnittstellenoberfläche hin gerichtet;
   einen Fotoempfänger (906A, 906B, 906C, 906D), um den Laser herum positioniert und parallel zu der zweiten Schnittstellenoberfläche; und
   einen Prozessor (407), aufweisend einen Speicher, wirkverbunden mit dem Laser und dem Fotoempfänger.

2. Implantierbares Impulsgeneratorsystem nach Anspruch 1, wobei der Laser ein VCSEL ist oder

   wobei der parabolische Umlenker eine Kollimatorlinse (826), die auf der optischen Achse zentriert ist, einschließt oder
   wobei der Laser auf dem Chipstapel durch ein optisches Fenster befestigt ist, vorzugsweise wobei der Laser mit dem Prozessor durch eine metallische Leiterbahn elektrisch verbunden ist, befestigt auf dem optischen Fenster, oder
   wobei das Gehäuse ferner umfasst:

   einen metallischen ersten Abschnitt, der eine Kopfstückausbuchtung ausbildet;
   ein Kopfstück, das das Leitungshalteloch enthält, befestigt in der Kopfstückausbuchtung; und
   einen keramischen zweiten Abschnitt, hermetisch versiegelt mit dem metallischen ersten Abschnitt.

3. Implantierbares Impulsgeneratorsystem nach Anspruch 1, ferner umfassend:
   einen Satz torusförmiger elektrischer Kontakte (812, 814), befestigt in dem Leitungshalteloch, axial ausgerichtet zu der optischen Achse.

4. Implantierbares Impulsgeneratorsystem nach Anspruch 3, ferner umfassend:

   eine flexible Leitung (422A, 422B, 424A, 424B), die in dem Leitungshalteloch befestigt ist;
   eine zentral angeordnete optische Faser (1002), die in der flexiblen Leitung einstückig ausgebildet ist, koaxial zu der optischen Achse;
   einen Satz von zylindrischen Kontakten (1108A, 1108B, 1108C, 1108D, 1108E, 1108F, 1108G, 1108H), befestigt an einer Außenoberfläche der flexiblen Leitung, elektrisch verbunden mit dem Satz von torusförmigen elektrischen Kontakten; und
   einen Satz zylindrischer Elektroden (1106A, 1106B, 1106C, 1106D, 1106E, 1106F, 1106G, 1106H), befestigt an der Außenoberfläche der flexiblen Leitung, elektrisch verbunden mit dem Satz von zylindrischen Kontakten.

5. Implantierbares Impulsgeneratorsystem nach Anspruch 4, wobei die flexible Leitung ferner umfasst:

   ein Längsstilettlumen (1131A, 1131B, 1131C, 1131D, 1131E, 1131F, 1131G, 1131H), benachbart und parallel zu der zentral angeordneten optischen Faser radial angeordnet, vorzugsweise wobei das Längsstilettlumen in einem Stilettanschlag (1154) distal endet, oder
   wobei die flexible Leitung umfasst ferner:
   eine transparente optische Übertragungsspitze (1109), einstückig ausgebildet mit der zentral angeordneten optischen Faser, vorzugsweise wobei die transparente optische Übertragungsspitze ferner einen zentral angeordneten röntgenopaken Marker (1152) umfasst.

6. Implantierbares Impulsgeneratorsystem nach Anspruch 4, ferner umfassend:
   einen Satz von Anweisungen, resident in dem Speicher, die bei Ausführung das implantierbare Impulsgeneratorsystem veranlassen zum:

   Generieren eines ersten Übertragungsstrahls mit einer ersten Wellenlänge aus dem Laser;
   Senden des ersten Übertragungsstrahls durch den parabolischen Umlenker in die zentral angeordnete optische Faser hinein;
   Empfangen eines ersten Empfangsstrahls, von der zentral angeordneten optischen Faser, durch den parabolischen Umlenker, einfallend auf den Fotoempfänger;

Generieren einer Variationsvariablen aus dem ersten Empfangsstrahl;

Erzeugen eines modulierten Stimulationssignals basierend auf der Variationsvariablen; und

Senden des modulierten Stimulationssignals an den Satz von zylindrischen Elektroden, vorzugsweise wobei der Satz von Anweisungen weitere Anweisungen umfasst, resident in dem Speicher, die bei Ausführung das implantierbare Impulsgeneratorsystem veranlassen zum:

Generieren eines zweiten Übertragungsstrahls mit einer zweiten Wellenlänge aus dem Laser;

Senden des zweiten Übertragungsstrahls durch den parabolischen Umlenker in die zentral angeordnete optische Faser hinein;

Empfangen eines zweiten Empfangsstrahls, von der zentral angeordneten optischen Faser, durch den parabolischen Umlenker, einfallend auf den Fotoempfänger;

Generieren eines Kompensationswerts aus dem zweiten Empfangsstrahl; und

Ändern des modulierte Stimulationssignals basierend auf dem Kompensationswert.

7. Implantierbares Impulsgeneratorsystem, umfassend:

ein Gehäuse (401);

eine erste Kopfstückausbuchtung (604), ausgebildet in dem Gehäuse;

eine zweite Kopfstückausbuchtung (606), diametral angeordnet zu der ersten Kopfstückausbuchtung, ausgebildet in dem Gehäuse;

eine erste Kopfstückbaugruppe (502), befestigt in der ersten Kopfstückausbuchtung, aufweisend einen ersten Leitungshaltekanal (523A) und einen zweiten Leitungshaltekanal (523B);

eine zweite Kopfstückbaugruppe (504), befestigt in der zweiten Kopfstückausbuchtung, aufweisend einen dritten Leitungshaltekanal (525A) und einen vierten Leitungshaltekanal (525B);

eine erste elektro-optische Leitung (422A), aufweisend eine erste optische Achse, positioniert in dem ersten Leitungshaltekanal;

eine zweite elektro-optische Leitung (422B), aufweisend eine zweite optische Achse, positioniert in dem zweiten Leitungshaltekanal;

eine dritte elektro-optische Leitung (424A), aufweisend eine dritte optische Achse, positioniert in dem dritten Leitungshaltekanal;

eine vierte elektro-optische Leitung (424B), aufweisend eine vierte optische Achse, positioniert in dem vierten Leitungshaltekanal;

einen ersten parabolischen Umlenker, zentriert auf der ersten optischen Achse, optisch gekoppelt an die erste elektro-optische Leitung;

einen zweiten parabolischen Umlenker, zentriert auf der zweiten optischen Achse, optisch gekoppelt an die zweite elektro-optische Leitung;

einen dritten parabolischen Umlenker (822), zentriert auf der dritten optischen Achse, optisch gekoppelt an die dritte elektro-optische Leitung;

einen vierten parabolischen Umlenker (820), zentriert auf der vierten optischen Achse, optisch gekoppelt an die vierte elektro-optische Leitung;

einen ersten Chipstapel (718), aufweisend einen ersten senkrecht ausgerichteten Laser, umgeben von einer ersten Fotodiode, optisch gekoppelt an den ersten parabolischen Umlenker;

der erste Chipstapel aufweisend einen zweiten senkrecht ausgerichteten Laser, umgeben von einer zweiten Fotodiode, optisch gekoppelt mit dem zweiten parabolischen Umlenker;

einen zweiten Chipstapel (720), aufweisend einen dritten senkrecht ausgerichteten Laser (850), umgeben von einer dritten Fotodiode (906A), optisch gekoppelt an den dritten parabolischen Umlenker; und

der zweite Chipstapel aufweisend einen vierten senkrecht ausgerichteten Laser (852), umgeben von einer vierten Fotodiode (906B), optisch gekoppelt an den vierten parabolischen Umlenker.

8. Implantierbares Impulsgeneratorsystem nach Anspruch 7, wobei die erste elektro-optische Leitung ferner umfasst:

eine optische Faser (1002), koaxial positioniert zu der ersten optischen Achse;

einen Satz von elektrischen Kontakten (1108A, 1108B, 1108C, 1108D, 1108E, 1108F, 1108G, 1108H), befestigt auf einer proximalen Oberfläche der ersten elektro-optischen Leitung; und

einen Satz von Elektroden (1106A, 1106B, 1106C, 1106D, 1106E, 1106F, 1106G, 1106H), befestigt an einer distalen Oberfläche der ersten elektro-optischen Leitung, elektrisch verbunden mit dem Satz von elektrischen Kontakten.

9. Implantierbares Impulsgeneratorsystem nach Anspruch 8, wobei die erste Kopfstückausbuchtung ferner umfasst:

einen Satz von festen Kontakten (812), starr in dem ersten Leitungshaltekanal positioniert und wirkverbunden mit einem elektro-optischen Signalgenerator;
wobei der elektro-optische Signalgenerator programmiert ist zum:

Senden eines ersten Übertragungsstrahls einer ersten Wellenlänge von dem ersten senkrecht ausgerichteten Laser in den ersten parabolischen Umlenker und die optische Faser;
Empfangen eines ersten Rücksignals von der ersten Fotodiode basierend auf einem ersten Empfangsstrahl;
Generieren eines Stimulationssignals basierend auf dem ersten Rücksignal; und
Senden des Stimulationssignals an den Satz von festen Kontakten zur Übertragung an den Satz von Elektroden.

10. Implantierbares Impulsgeneratorsystem nach Anspruch 9, wobei der elektro-optische Signalgenerator ferner programmiert ist zum:

Senden eines zweiten Übertragungsstrahls einer zweiten Wellenlänge von dem ersten senkrecht ausgerichteten Laser in den ersten parabolischen Umlenker und die erste elektro-optische Leitung;
Empfangen eines zweiten Empfangsstrahls an der ersten Fotodiode;
Generieren eines Kompensationswerts aus dem zweiten Empfangsstrahl; und Modifizieren des Stimulationssignals basierend auf dem Kompensationswert, vorzugsweise, wobei:

die erste Wellenlänge zwischen etwa 700 Nanometer und etwa 800 Nanometer liegt; und
die zweite Wellenlänge zwischen etwa 400 Nanometer und etwa 500 Nanometer oder zwischen etwa 520 Nanometer und etwa 532 Nanometer liegt.

11. Implantierbares Impulsgeneratorsystem nach Anspruch 9, wobei:

der erste Chipstapel ferner eine zweite Fotodiode umfasst;
wobei der elektro-optische Signalgenerator ferner programmiert ist zum:
Normalisieren eines ersten Versorgungsstroms an die erste Fotodiode und eines zweiten Versorgungsstroms an die zweite Fotodiode, vorzugsweise wobei der Schritt des Normalisierens ferner umfasst:

Reduzieren des ersten Versorgungsstroms, wenn der erste Versorgungsstrom größer als der zweite Versorgungsstrom ist; und
Reduzieren des zweiten Versorgungsstroms, wenn der zweite Versorgungsstrom größer als der erste Versorgungsstrom ist, oder
der elektro-optische Signalgenerator ferner programmiert ist zum:
Korrigieren einer zeitbasierten Variation in dem ersten Versorgungsstrom, vorzugsweise wobei der Schritt des Korrigierens ferner umfasst:
Ableiten einer Differenz zwischen einem anfänglichen Versorgungsstrom zu der ersten Fotodiode und dem ersten Versorgungsstrom.

12. Implantierbares Impulsgeneratorsystem nach Anspruch 7, wobei der erste parabolische Umlenker ferner umfasst:

eine Kollimatorlinse, zentriert auf der ersten optischen Achse; und
eine parabolische Oberfläche zum Reflektieren eines Übertragungsstrahls zu der ersten elektro-optischen Leitung hin und eines Empfangsstrahls zu der ersten Fotodiode hin, vorzugsweise wobei die parabolische Oberfläche eine reflektierende Beschichtung einschließt, oder ferner umfassend:

eine transparente Abdeckplatte, zwischen dem ersten senkrecht ausgerichteten Laser und dem ersten Chipstapel, die den ersten senkrecht ausgerichteten Laser benachbart zu dem ersten parabolischen Umlenker positioniert,
vorzugsweise ferner umfassend:
ein Fenster, abgedichtet an dem Gehäuse, zwischen dem ersten senkrecht ausgerichteten Laser und dem ersten parabolischen Umlenker.

## Revendications

1. Système générateur d'impulsions implantable comprenant :

un boîtier (401) ;
un trou de retenue de conducteur (523A, 523B, 525A, 525B), positionné longitudinalement dans le boîtier, ayant un axe optique ;
un redirecteur parabolique (820, 822), ayant une première surface d'interface perpendiculaire à une seconde surface d'interface reliée par une surface parabolique, focalisé sur l'axe optique ;
un empilement de puces (718, 720), adjacent au redirecteur parabolique, perpendiculaire à l'axe optique et parallèle à la seconde surface d'interface ;

un laser (850, 852), fixé sur l'empilement de puces, dirigé verticalement en direction de la seconde surface d'interface ;

un photorécepteur (906A, 906B, 906C, 906D), positionné autour du laser et parallèle à la seconde surface d'interface ; et

un processeur (407), ayant une mémoire, connecté fonctionnellement au laser et au photorécepteur.

2.  Système générateur d'impulsions implantable selon la revendication 1, dans lequel le laser est un VCSEL, ou

dans lequel le redirecteur parabolique comporte une lentille de collimation (826) centrée sur l'axe optique, ou

dans lequel le laser est fixé sur l'empilement de puces par une fenêtre optique, de préférence dans lequel le laser est connecté électriquement au processeur par un tracé métallique, fixé sur la fenêtre optique, ou

dans lequel le boîtier comprend en outre :

une première section métallique formant un logement d'embase ;

une embase, contenant le trou de retenue de conducteur, fixée dans le logement d'embase ; et

une seconde section céramique, hermétiquement scellée à la première section métallique.

3.  Système générateur d'impulsions implantable selon la revendication 1, comprenant en outre :
un ensemble de contacts électriques toroïdaux (812, 814), fixés dans le trou de retenue de conducteur, alignés axialement sur l'axe optique.

4.  Système générateur d'impulsions implantable selon la revendication 3, comprenant en outre :

un conducteur flexible (422A, 422B, 424A, 424B), fixé dans le trou de retenue de conducteur ;

une fibre optique disposée de manière centrale (1002), formée en une seule pièce dans le conducteur flexible, coaxiale par rapport à l'axe optique ;

ensemble de contacts cylindriques (1108A, 1108B, 1108C, 1108D, 1108E, 1108F, 1108G, 1108H), fixés sur une surface extérieure du conducteur flexible, connectés électriquement à l'ensemble de contacts électriques toroïdaux ; et

un ensemble d'électrodes cylindriques (1106A, 1106B, 1106C, 1106D, 1106E, 1106F, 1106G, 1106H), fixées sur la surface extérieure du conducteur flexible, connectées électriquement à l'ensemble de contacts cylindriques.

5.  Système générateur d'impulsions implantable selon la revendication 4, dans lequel le conducteur flexible comprend en outre :

une lumière de stylet longitudinale (1131A, 1131B, 1131C, 1131D, 1131E, 1131F, 1131G, 1131H), disposée radialement adjacente et parallèle à la fibre optique disposée de manière centrale, de préférence dans lequel la lumière de stylet longitudinale se termine distalement en un arrêt de stylet (1154), ou

le conducteur flexible comprend en outre :

une pointe de transmission optique transparente (1109), formée en une seule pièce avec la fibre optique disposée de manière centrale, de préférence dans lequel la pointe de transmission optique transparente comprend en outre un marqueur radio-opaque disposé de manière centrale (1152).

6.  Système générateur d'impulsions implantable selon la revendication 4, comprenant en outre :
un ensemble d'instructions, se trouvant dans la mémoire, qui lorsqu'elles sont exécutées amènent le système générateur d'impulsions implantable à :

générer un premier rayon de transmission, à une première longueur d'onde, à partir du laser ;

envoyer le premier rayon de transmission à travers le redirecteur parabolique le long de la fibre optique disposée de manière centrale ;

recevoir un premier rayon de réception, en provenance de la fibre optique disposée de manière centrale, à travers le redirecteur parabolique, incident sur le photorécepteur ;

générer une variable de variation à partir du premier rayon de réception ;

créer un signal de stimulation modulé en fonction de la variable de variation ; et

envoyer le signal de stimulation modulé à l'ensemble d'électrodes cylindriques, de préférence dans lequel l'ensemble d'instructions comprend d'autres instructions, se trouvant dans la mémoire, qui lorsqu'elles sont exécutées amènent le système générateur d'impulsions implantable à :

générer un second rayon de transmission, d'une seconde longueur d'onde, à partir du laser ;

envoyer le second rayon de transmission à travers le redirecteur parabolique le long de la fibre optique disposée de manière centrale ;

recevoir un second rayon de réception, en provenance de la fibre optique disposée de manière centrale, à travers le redirecteur parabolique, incident sur le photorécepteur ;

générer une valeur de compensation à partir du second rayon de réception ; et

modifier le signal de stimulation modulé en fonction de la valeur de compensation.

7. Système générateur d'impulsions implantable comprenant :

un boîtier (401) ;

un premier logement d'embase (604), formé dans le boîtier ;

un second logement d'embase (606), disposé diamétralement par rapport au premier logement d'embase, formé dans le boîtier ;

un premier ensemble embase (502), fixé dans le premier logement d'embase, ayant un premier canal de retenue de conducteur (523A) et un deuxième canal de retenue de conducteur (523B) ;

un second ensemble embase (504), fixé dans le second logement d'embase, ayant un troisième canal de retenue de conducteur (525A) et un quatrième canal de retenue de conducteur (525B) ;

un premier conducteur électro-optique (422A), ayant un premier axe optique, positionné dans le premier canal de retenue de conducteur ;

un deuxième conducteur électro-optique (422B), ayant un deuxième axe optique, positionné dans le deuxième canal de retenue de conducteur ;

un troisième conducteur électro-optique (424A), ayant un troisième axe optique, positionné dans le troisième canal de retenue de conducteur ;

un quatrième conducteur électro-optique (424B), ayant un quatrième axe optique, positionné dans le quatrième canal de retenue de conducteur ;

un premier redirecteur parabolique, centré sur le premier axe optique, couplé optiquement au premier conducteur électro-optique ;

un deuxième redirecteur parabolique, centré sur le deuxième axe optique, couplé optiquement au deuxième conducteur électro-optique ;

un troisième redirecteur parabolique (822), centré sur le troisième axe optique, couplé optiquement au troisième conducteur électro-optique ;

un quatrième redirecteur parabolique (820), centré sur le quatrième axe optique, couplé optiquement au quatrième conducteur électro-optique ;

un premier empilement de puces (718), ayant un premier laser orienté perpendiculairement, entouré par une première photodiode, couplé optiquement au premier redirecteur parabolique ;

le premier empilement de puces, ayant un deuxième laser orienté perpendiculairement, entouré par une deuxième photodiode, couplé optiquement au deuxième redirecteur parabolique ;

un second empilement de puces (720), ayant un troisième laser orienté perpendiculairement (850), entouré par une troisième photodiode (906A), couplé optiquement au troisième redirecteur parabolique ; et

le second empilement de puces, ayant un quatrième laser orienté perpendiculairement (852), entouré par une quatrième photodiode (906B), couplé optiquement au quatrième redirecteur parabolique.

8. Système générateur d'impulsions implantable selon la revendication 7, dans lequel le premier conducteur électro-optique comprend en outre :

une fibre optique (1002), positionnée coaxialement par rapport au premier axe optique ;

un ensemble de contacts électriques (1108A, 1108B, 1108C, 1108D, 1108E, 1108F, 1108G, 1108H), fixés sur une surface proximale du premier conducteur électro-optique ; et

un ensemble d'électrodes (1106A, 1106B, 1106C, 1106D, 1106E, 1106F, 1106G, 1106H), fixées sur une surface distale du premier conducteur électro-optique, connectées électriquement à l'ensemble de contacts électriques.

9. Système générateur d'impulsions implantable selon la revendication 8, dans lequel le premier logement d'embase comprend en outre :

un ensemble de contacts fixes (812), positionnés de façon rigide dans le premier canal de retenue de conducteur, et connecté fonctionnellement à un générateur de signal électro-optique ;

dans lequel le générateur de signal électro-optique est programmé pour :

envoyer un premier rayon de transmission, d'une première longueur d'onde, à partir du premier laser orienté perpendiculairement dans le premier redirecteur parabolique et la fibre optique ;

recevoir un premier signal de retour en provenance de la première photodiode, en fonction d'un premier rayon de réception ;

générer un signal de stimulation en fonction du premier signal de retour ; et

envoyer le signal de stimulation à l'ensemble de contacts fixes, pour transmission à l'ensemble d'électrodes.

10. Système générateur d'impulsions implantable selon la revendication 9, dans lequel le générateur de signal électro-optique est programmé en outre pour :

envoyer un second rayon de transmission, d'une seconde longueur d'onde, à partir du premier laser orienté perpendiculairement dans le premier redirecteur parabolique et le premier conducteur électro-optique ;
recevoir un second rayon de réception au niveau de la première photodiode ;
générer une valeur de compensation à partir du second rayon de réception ; et modifier le signal de stimulation en fonction de la valeur de compensation, de préférence, dans lequel :

la première longueur d'onde est comprise entre environ 700 nanomètres et environ 800 nanomètres ; et
la seconde longueur d'onde est comprise entre environ 400 nanomètres et environ 500 nanomètres ou entre environ 520 nanomètres et environ 532 nanomètres.

11. Système générateur d'impulsions implantable selon la revendication 9, dans lequel :

le premier empilement de puces comprend en outre une deuxième photodiode ;
dans lequel le générateur de signal électro-optique est programmé en outre pour :
normaliser un premier courant d'alimentation vers la première photodiode et un second courant d'alimentation vers la deuxième photodiode, de préférence dans lequel l'étape de normalisation comprend en outre :

la réduction du premier courant d'alimentation si le premier courant d'alimentation est supérieur au second courant d'alimentation ; et
la réduction du second courant d'alimentation si le second courant d'alimentation est supérieur au premier courant d'alimentation, ou
le générateur de signal électro-optique est programmé en outre pour :
compenser une variation temporelle dans le premier courant d'alimentation, de préférence dans lequel l'étape de correction comprend en outre :
la dérivation d'une différence entre un courant d'alimentation initial vers la première photodiode et le premier courant d'alimentation.

12. Système générateur d'impulsions implantable selon la revendication 7, dans lequel le premier redirecteur parabolique comprend en outre :

une lentille de collimation centrée sur le premier axe optique ; et
une surface parabolique, permettant de réfléchir un rayon de transmission en direction du premier conducteur électro-optique et un rayon de réception en direction de la première photodiode, de préférence dans lequel la surface parabolique comporte un revêtement réfléchissant, ou
comprenant en outre :

une plaque de recouvrement transparente, entre le premier laser orienté perpendiculairement et le premier empilement de puces, positionnant le premier laser orienté perpendiculairement adjacent au premier redirecteur parabolique,
de préférence comprenant en outre :
une fenêtre, scellée au boîtier, entre le premier laser orienté perpendiculairement et le premier redirecteur parabolique.

**FIG. 1**
(PRIOR ART)

FIG. 2
(PRIOR ART)

FIG. 3
(PRIOR ART)

FIG. 4

FIG. 5

EP 4 376 943 B1

FIG. 6

FIG. 7

FIG. 8

FIG. 9

FIG. 10

EP 4 376 943 B1

FIG. 11

FIG. 12

1300

1302 — START

1304 — SET CHANNEL

1306 — ACTIVATE LASER

1307 — NORMALIZE SOURCE EMISSION

1308 — CORRECT FOR SOURCE POWER VARIATION

1309 — POLL PHOTODIODE

1310 — CALCULATE STIMULATION SIGNAL CURRENT

1312 — DERIVE COMPENSATED STIMULATION CURRENT VALUE

1313 — SEND COMPENSATED STIMULATION SIGNAL

1314 — POLL COMMUNICATION CIRCUIT

1316 — SHUT DOWN SIGNAL PRESENT?

NO — GO TO NEXT CHANNEL — 1320

YES

1318 — STOP

FIG. 13

1312

1402 — ( START )

1404 — SEND BLUE SIGNAL

1406 — MEASURE BLUE SIGNAL RETURN

1408 — CALCULATE REFLECTION COMPENSATION VALUE

1410 — GET CALCULATED STIMULATION CURRENT VALUE

1412 — CALCULATE COMPENSATED STIMULATION CURRENT VALUE

1414 — RETURN $I_{new}$

1416 — ( END )

FIG. 14

1307

1502 — ( START )

1504 — READ OUTPUT MONITOR (1)

1506 — READ OUTPUT MONITOR (2)

1508 — IS (1) > (2)? — NO

YES

1512 — CALCULATE DIFFERENCE

1513 — REDUCE (1)

IS (2) > (1)? — NO

1514 — YES

1515 — CALCULATE DIFFERENCE

1516 — REDUCE (2)

1518 — RETURN POWER LEVEL (1) AND (2)

1520 — ( END )

FIG. 15

1308

1602 — ( START )

1604 — GET INITIAL POWER LEVEL

1606 — ACTIVATE LASER

1608 — POLL MONITOR PHOTODIODE

1610 — STORE MONITOR PHOTODIODE LEVEL

1612 — DERIVE DIFFERENCE BETWEEN MONITOR POWER LEVEL AND INITIAL POWER LEVEL

1614 — STORE DIFFERENCE

1616 — CALCULATE CORRECTED POWER LEVEL

1618 — RETURN CORRECTED POWER LEVEL

1620 — ( END )

FIG. 16

**EP 4 376 943 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10035019 B
- US 9656097 B, Wolf II
- US 7742817 B, Malinowski
- US 20210001114 A, Wolf, II,
- US 20180154152 A, Chabrol
- US 9550063 B, Wolf II